# EUROPEAN PATENT SPECIFICATION

(11) **EP 0 562 853 B1**
(45) Date of publication and mention of the grant of the patent: **05.06.1996**
(21) Application number: 93302288.1
(22) Date of filing: 25.03.1993
(51) Int. Cl.: A61K 31/71, A61K 31/445, A61K 31/57, A61K 31/535, A61K 38/13

(54) **29-Demethoxyrapamycin for inducing immunosuppression**
29-Demethoxyrapamycin zur Induktion der Immunosuppression
29-Demethoxyrapamycin pour induire l'immunosuppression

(30) Priority: 27.03.1992 US 858923
(43) Date of publication of application: 29.09.1993
(73) Proprietor: AMERICAN HOME PRODUCTS CORPORATION, Madison, New Jersey 07940-0874 (US)
(72) Inventor: Sehgal, Suren Nath, Princeton, NJ (US); Armstrong, Jay Joseph, Bensalem, PA (US); Eng, Chee Ping, Princeton Junction, NJ (US)
(74) Representative: Wileman, David Francis, Dr.

(56) References cited:
- EP-A- 475 577
- EP-A- 0 184 162
- EP-A- 0 401 747
- WO-A-92/14737
- TRANSPLANTATION PROCEEDINGS, vol. 23, no. 1, February 1991; S.M. STEPKOWSKI et al., pp. 507-508
- JOURNAL OF ANTIBIOTICS, vol. 37, no. 10, 1984; C.P. ENG et al., pp. 1231-1237

## Description

This invention relates to a method of inducing immunosuppression, in particular to a new use of 29-demethoxyrapamycin.

Recently, it has been recognized that numerous diseases or disorders can be prevented or treated through the use of immunosuppressive agents. In particular, through the use of immunosuppressive agents, organ transplantation has become a successful method of treating many serious disease states that otherwise might be fatal. What was once only an experimental procedure used in emergency life-threatening situations, is now used early in the care of patients with severe chronic diseases. Currently, the kidney, heart, lung, liver, bone marrow, pancreas (islet cells), cornea, small bowel, and skin are among allografts that are routinely performed. Additionally, xenografts are now performed using porcine heart valves.

Despite the advances made in the field of organ allografting, transplantation rejection remains the predominate factor leading to graft failure. Overall, graft rejection is a complex process involving the immune system. This process is briefly outlined below. Rejection appears to be initiated by blood borne antigen presenting cells (dendritic cells and monocytes expressing class II MHC molecules) of the allograft which migrate from the allograft. Antigen recognition and production of IL-1 by the antigen presenting cells causes the activation of CD4⁺ T-cells thereby initiating an immune response leading to the ultimate graft rejection. Activated CD4⁺ T-cells produce IL-2 which is a growth factor essential to the activation of both CD8⁺ T-cells and B-cells. Clonal proliferation and maturation of alloantigen reactive cells leads to the production of effector T-cells (cytotoxic CD8⁺ T-cells and CD4⁺ T-cells) which migrate from the host lymphoid tissue and infiltrate the graft tissue. Infiltration involves initial adherence of the T-lymphocytes to vascular endothelium, transmigration through the vascular wall, migration within the graft, selective retention of activated cells within the graft, and local proliferation of cells. Antigen presenting graft cells are destroyed directly by cytotoxic CD8⁺ T-cells. Additionally, CD4⁺ T-cells produce other lymphokines such as interferon-γ (IFN-γ), IL-4, and IL-5 which also contribute to graft destruction. IFN-γ induces increased expression of HLA-A, -B, and -DR on graft tissue malting it more vulnerable to effector mechanisms. IFN-γ also activates macrophages to initiate a delayed hypersensitivity reaction causing nonspecific damage to the graft. IL-4 and IL-5 are implicated in inducing antibody production by plasma cells leading to antibody mediated damage of the graft. [Hutchinson, I., Transplantation 3: 722 (1991); Garovoy, M.R., Basic and Clinical Immunology, ed. Stites, 7th ed., 747 (1991)].

Currently, allograft rejection is controlled using agents which suppress the immune response such as prednisone, methylprednisolone, azathioprine, cyclophosphamide, cyclosporine, antilymphocyte globulin, monoclonal antibodies, and irradiation. Of the chemotherapeutic agents currently used cyclosporin A is the most powerful and most frequently used, but has the unsatisfactory side-effect of nephrotoxicity in man, which can lead to structural renal damage.

Recently, rapamycin, a macrocyclic triene antibiotic produced by Streptomyces hygroscopicus [U.S. Patent 3,929,992] has been shown to prevent the formation of humoral (IgE-like) antibodies in response to an albumin allergic challenge [Martel, R., Can. J. Physiol. Pharm. 55: 48 (1977)], inhibit murine T-cell activation [Staruch, M., FASEB 3: 3411 (1989)], prolong survival time of organ grafts in histoincompatible rodents [Morris, R., Med. Sci. Res. 17: 877 (1989)], and inhibit transplantation rejection in mammals [Calne, R., European Patent Application 401,747]. FK-506, a tricyclomacrolide antibiotic produced by Streptomyces tsukubaensis, has also been shown to have immunosuppressive activity and preyent transplantation rejection. [U.S. Patent 4,894,366; Armitage, J., Transplantation 52: 164 (1989); Fung, J.J., Transplantation Proc. 23: 1902 (1991); Murase, N., Surgery 110: 87 (1991)].

The immunosuppressive agents rapamycin, cyclosporin A, and/or FK-506 have also been shown to be useful in treating diseases and conditions involving the immune system. These include autoimmune diseases, immunoinflammatory disorders of the skin, eye, kidney, and bowel, pulmonary inflammation, and restenosis following balloon catheterization. [U.S. Patent 4,894,366; U.S. Patent 5,078,999; U.S. Patent 5,080,899; U.S. Patent 5,091,389; Berden, J., Scand. J. Immunol. 24: 405 (1986); Mounts, J., J. Immunol. 138: 157 (1987); Takabayshi, K., Clin. Immunol. Immounpath. 51: 110 (1989); Ellis, JAMA 256: 3110 (1986); G. Feutren, Lancet 119: (1986); J. Dupre, Diabetes 37: 1574, (1988); C.R. Stiller, Science 223: 1362 (1984); R. Lipton, Diabetes Care, 13: 776 (1990); K. Wilson, Annu. Rev. Med. 41: 497 (1990); N. Murase, Diabetes 39: 1584 (1990); K. Kurasawa, Clin. Immun. Immunopath. 57: 274 (1990); J. Miyagawa, Diabetologia 33: 503 (1990); Cur. Eye. Res. 9: 749 (1990); Fifth Int. Conf. Inflam. Res. Assoc. 121 (1990); Morris, R. J. Heart Lung Transplant. 11 (pt. 2): 197, (1992)].

29-Demethoxyrapamycin a macrocyclic triene antibiotic produced by Streptomyces hygroscopicus, NRRL 5491 [US Patent 4,375,464;AY 24668] has been shown to have antifungal activity against Candida albicans, marginal activity against P388 lymphocytic leukemia and no activity against B16 melanocarcinoma and Colon 38 solid tumor . [Sehgal, S J Antibiotics 36: 351 (1983)]. 29-Demethoxyrapamycin may also be referred to as 32-demethoxyrapamycin under C.A. nomenclature.

This invention provides use of 29-demethoxyrapamycin in the preparation of a medicament for inducing immunosuppression in a mammal. This invention also provides a method of inducing immunosuppression in a mammal in need thereof by administering an immunosuppressive effective amount of 29-demethoxyrapamycin to said mammal orally, parenterally, intranasally, intrabronchially, transdermally, or rectally.

In a preferred embodiment, this invention provides a method of preventing or treating organ or tissue transplantation rejection in a mammal in need thereof by administering an antirejection effective amount of 29-demethoxyrapamycin.

This invention also provides a method of inducing immunosuppression in a mammal by administering an immunosuppressive effective amount of a combination of 29-demethoxyrapamycin and one or more other antirejection chemotherapeutic agents to said mammal orally, parenterally, intranasally, intrabronchially, transdermally, or rectally. Such other antirejection chemotherapeutic agents include, but are not limited to azathioprine, corticosteroids, such as prednisone and methylprednisolone, cyclophosphamide, rapamycin, cyclosporin A, FK-506, OKT-3, and ATG. By combining 29-demethoxyrapamycin with such other drugs or agents for inducing immunosuppression, the toxicity of the latter may be reduced in that lesser amounts of such agents are required to induce immunosuppression. The basis for such combination therapy was established by Stepkowski whose results showed that the use of a combination of rapamycin and cyclosporin A at subtherapeutic doses significantly prolonged heart allograft survival time. [Transplantation Proc. 23: 507 (1991)].

In a preferred embodiment, the combination of 29-demethoxyrapamycin and one or more other antirejection chemotherapeutic agents provides a method of preventing or treating organ or tissue transplantation rejection in a mammal in need thereof.

This invention also provides a composition for inducing immunosuppression which comprises an immunosuppressive effective amount of 29-demethoxyrapamycin and a pharmaceutically acceptable carrier. This invention also provides a composition for preventing or treating transplantation rejection comprising a antirejection effective amount of 29-demethoxyrapamycin and a pharmaceutically acceptable carrier. In another embodiment of this invention, the compositions each additionally contain one or more antirejection chemotherapeutic agents as defined above.

Transplantation rejection includes both host versus graft disease and graft versus host disease. Preventing includes the prophylactic prevention of transplantation rejection in a susceptible mammal. Treating includes arresting the progression of transplantation rejection, maintaining transplant viability, as well as reversing transplantation rejection. Therefore, it is contemplated that 29-demethoxyrapamycin, alone or in combination with another antirejection chemotherapeutic agent, will be administered prior to and/or following organ or tissue transplantation into said mammal. It will be recognized by one skilled in the art that compounds, drugs, agents, and the like, may be administered to such mammal, for example a human, for an indefinite post-transplant period, and in some instances, for the lifetime of such mammal, provided that such mammal is tolerating the compound, drug, or agent reasonably well without serious side effects.

As such 29-demethoxyrapamycin, alone or in combination with another antirejection chemotherapeutic agent, is useful in preventing or treating organ or tissue transplantation rejection in following organ or tissue allograft and xenograft procedures, such as kidney, heart, liver, lung, bone marrow, pancreas (islet cells), cornea, small bowel, and skin allografts, and heart valve xenografts, and the like. The use of 29-demethoxyrapamycin, alone or in combination with another antirejection chemotherapeutic agent, to prevent or treat transplantation rejection of other organs, tissue, or cells will be apparent to one skilled in the art based on this disclosure.

The ability of 29-demethoxyrapamycin to induce immunosuppression was established in two in vivo standard pharmacological test procedures. The first in vivo procedure evaluated the survival time of a pinch skin graft. The second test procedure measured survival the time of a transplanted heart allograft. The procedures used and the results obtained are briefly described below.

The first in vivo test procedure is designed to determine the survival time of pinch skin graft from male BAB/c donors transplanted to male C₃H(H-2K) recipients. The method is adapted from Billingham R.E. and Medawar P.B., J. Exp. Biol. 28:385-402, (1951). Briefly, a pinch skin graft from the donor was grafted on the dorsum of the recipient as a allograft, and an isograft was used as control in the same region. The recipients were treated with either varying concentrations of 29-demethoxyrapamycin intraperitoneally or orally. Cyclosporin A and rapamycin were used as test controls. Untreated recipients serve as rejection control. The graft was monitored daily and observations were recorded until the graft became dry and formed a blackened scab. This was considered as the rejection day. The mean graft survival time (number of days ± S.D.) of the drug treatment group was compared with the control group. The following table shows the results that were obtained. Results are expressed as the mean survival time in days.

| SKIN GRAFT SURVIVAL TIME | | | |
|---|---|---|---|
| Compound | Route | Dose | Survival Time ± S.D. (days) |
| No Treatment | | | 7.67 ± 0.33 |
| 29-Demethoxyrapamycin | i.p. | 4 mg/kg | 10.00 ± 0.26 |
| | | 1 mg/kg | 9.60 ± 0.24 |
| | | 0.25 mg/kg | 8.67 ± 0.33 |
| | p.o. | 40 mg/kg | 10.50 ± 0.22 |
| | | 10 mg/kg | 9.50 ± 0.50 |
| | | 2.5 mg/kg | 9.33 ± 0.33 |
| Cyclosporin A | i.p. | 32 mg/kg | 10.17 ± 0.44 |
| | p.o. | 50 mg/kg | 9.83 ± 0.41 |
| Rapamycin | i.p. | 4 mg/kg | 11.67 ± 1.0 |
| | | 40 mg/kg | 13.88 ± 2.6 |

The results of this in vivo standard pharmacological test procedure showed that treatment 29-demethoxyrapamycin induced immunosuppression, and thereby prevented skin graft rejection as shown by the increased survival time of the skin graft for the mice treated with 29-demethoxyrapamycin as compared with the survival time of the skin graft in untreated control mice.

The ability of 29-demethoxyrapamycin to induce immunosuppression was further demonstrated in a heterotropic heart allograft standard pharmacological test procedure that emulates transplantation rejection that occurs in humans. The following briefly describes the procedure that was used. Male BN rat neonate donors (less than 5 days of age) were humanely sacrificed, the thymus was dissected away from the heart. All connections with the thoracic cavity were severed and the heart was removed from the chest cavity and placed in cooled RPMI media where all adherent fat and fascia were removed. The heart was bisected in half, along the midline from the apex to the root of the aorta, to generate two approximately equal halves each containing atrial and ventricular tissue. Recipient male Lewis rats were anesthetized with phenobarbital (50 mg/mL; i.p.), the left inner ear was swabbed with povidine iodine, and 1 mL RPMI was injected subcutaneously above the cartilage plate to produce a fluid filled sac. A stab incision was made to the sac, into which was inserted a single half heart fragment. The pocket was sealed with a single drop of Vet-Seal (3M Animal Care Products). Recipients were divided into groups of 10 rats each. One group was untreated and the second group was treated with 29-demethoxyrapamycin was administered at a dosage of 225 µg/day following the transplantation procedure until graft failure occurred. Administration was i.p., either by manual injection or via an Azlet osmotic pump that was implanted into the peritoneum of the recipient rat. Grafts were inspected for loss of cardiac activity on day 7 post-transplant and subsequently on alternate days. Graft survival time is defined as the post-transplant day on which the heart graft has lost all contractile activity by visual inspection and/or cardiac monitor. Individual rejection times were averaged to produce a mean survival time for each treated group.

The results show that treatment with 29-demethoxyrapamycin significantly (p <0.05) prolonged survival time of the heterotropic heart allograft compared with untreated controls. The mean survival time for the heterotropic heart allograft was 20.1 days in the 29-demethoxyrapamycin-treated group, whereas the heterotropic heart allograft was rejected in 9.65 days in untreated controls.

These results of these two in vivo standard pharmacological test procedures demonstrate that 29-demethoxyrapamycin is useful in inducing immunosuppression. As such, 29-demethoxyrapamycin is useful in preventing or treating transplantation rejection. More specifically, 29-demethoxyrapamycin is useful in preventing or treating transplantation rejection in following organ or tissue allograft and xenograft procedures, such as kidney, heart, lung, liver, bone marrow, pancreas (islet cells), cornea, small bowel, and skin allografts, and heart valve xenografts, and the like.

Based on its ability to induce immunosuppression, 29-demethoxyrapamycin is also considered to be useful in treating or preventing other diseases or disorders involving the immune system. These include, but are not limited to, autoimmune diseases and diseases of inflammation such as systemic lupus erythematosis, rheumatoid arthritis, diabetes mellitus, myasthenia gravis, aplastic anemia, pure red cell anemia, idiopathic thrombocytopenia, polychondritis, scleroderma, Wegener's granulomatosis, chronic active hepatitis, biliary cirrhosis, sarcioidosis, nephrotic syndrome, multiple sclerosis, Steven-Johnston syndrome, psoriasis, dermatitis, eczema, seborrhea, idiopathic sprue, Crohn's disease, inflammatory bowel disease, Graves ophthalmopathy, interstitial lung fibrosis, ocular inflammation, such as eye uveitis; pulmonary inflammation such as asthma, chronic obstructive pulmonary disease, emphysema, acute respiratory distress syndrome, bronchitis; and hyperproliferative vascular disorders such as restenosis and vascular occlusion, particularly following either biologically or mechanically mediated vascular injury leading to intimal smooth muscle cell proliferation.

When 29-demethoxyrapamycin, alone or in combination with another antirejection chemotherapeutic agent, is employed to induce immunosuppression, it can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compound can also be administered orally either in liquid or solid composition form.

29-Demethoxyrapamycin, alone or in combination with another antirejection chemotherapeutic agent, may be administered rectally in the form of a conventional suppository. For administration by intranasal or intrabronchial inhalation or insufflation, the compounds of this invention may be formulated into an aqueous or partially aqueous solution, which can then be utilized in the form of an aerosol. 29-Demethoxyrapamycin, alone or in combination with another antirejection chemotherapeutic agent, may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

29-Demethoxyrapamycin, alone or in combination with another antirejection chemotherapeutic agent, may be administered topically as a solution, cream, or lotion by formulation with pharmaceutically acceptable vehicles containing 0.1 - 5 percent, preferably 2%, of active compound.

The dosage requirements vary with the particular compositions employed, the route of administration, the severity of the symptoms presented and the particular subject being treated. Based on the results obtained in the standard pharmacological test procedures, projected daily intravenous dosages of 29-demethoxyrapamycin, when administered as the sole active compound, would be 0.001 - 25 mg/kg, preferably between 0.005 - 5 mg/kg, and more preferably between 0.01 - 0.5 mg/kg. Projected daily oral dosages of 29-demethoxyrapamycin, when administered as the sole active compound would be 0.005 - 75 mg/kg, preferably between 0.01 - 50 mg/kg, and more preferably between 0.05 - 10 mg/kg. When 29-demethoxyrapamycin is used in combination with another antirejection chemotherapeutic agent, it is projected that the dosage of 29-demethoxyrapamycin necessary to prevent or treat transplantation rejection will be reduced. It is also contemplated that the dosage of the other antirejection chemotherapeutic agent or agents, when used in combination with 29-demethoxyrapamycin, will also be less than required to achieve the same effect if used alone. The other antirejection chemotherapeutic agents can be administered continuously or intermittently with 29-demethoxyrapamycin.

Treatment will generally be initiated with small dosages less than the optimum dose of the compound. Thereafter the dosage is increased until the optimum effect under the circumstances is reached; precise dosages for oral, parenteral, intranasal, intrabronchial, transdermal, or rectal administration will be determined by the administering physician based on experience with the individual subject treated. In general, 29-demethoxyrapamycin, alone or in combination with another antirejection chemotherapeutic agent, is most desirably administered at a concentration that will generally afford effective results without causing any harmful or deleterious side effects.

In accordance with the above this invention also provides a product containing 29-demethoxyrapamycin and a anti-rejection chemotherapeutic agent as a combined preparation for simultaneous, separate or seqential use in inducing immunosuppression in a mammal.

## Claims

1. Use of 29-demethoxyrapamycin in the preparation of a medicament for inducing immunosuppression in a mammal.

2. Use of 29-demethoxyrapamycin in the preparation of a medicament for preventing or treating organ or tissue transplantation rejection in a mammal.

3. A use according to Claim 2 wherein the transplanted organ or tissue is selected from the group consisting of kidney, heart, liver, lung, bone marrow, pancreas (islet cells), cornea, small bowel, skin and heart valve.

4. A use according to any one of Claims 1 to 3 in which the medicament comprises a combination of 29-demethoxyrapamycin and one or more anti-rejection chemotherapeutic agents.

5. A use according to Claim 4 wherein the anti-rejection chemotherapeutic agent is selected from one or more of azathioprine, corticosteroids, cyclophosphamide, rapamycin, cyclosporin A, FK-506, OKT-3 and ATG.

6. A use according to any one of Claims 1 to 5 in which the medicament is adapted for administration orally, parenterally, intranasally, intrabronchially, transdermally or rectally.

7. A pharmaceutical composition which comprises a combination of 29-demethoxyrapamycin and one or more anti-rejection chemotherapeutic agents and a pharmaceutically acceptable carrier.

8. A product containing 29-demethoxyrapamycin and a anti-rejection chemotherapeutic agent as a combined preparation for simultaneous, separate or segential use in inducing immunosuppression in a mammal.

9. A composition or product according to Claim 7 or Claim 8 wherein the anti-rejection chemotherapeutic agent is selected from azathioprine, corticosteroids, cyclosporin A, FK-506, OKT-3 and ATG, or a combination of any of the foregoing.

## Patentansprüche

1. Verwendung von 29-Demethoxyrapamycin bei der Herstellung eines Medikaments zur Induktion einer Immunsuppression bei einem Säuger.

2. Verwendung von 29-Demethoxyrapamycin bei der Herstellung eines Medikaments zur Prävention oder Behandlung einer Organ- oder Gewebetransplantationsabstoßung bei einem Säuger.

3. Verwendung nach Anspruch 2, wobei das transplantierte Organ oder Gewebe ausgewählt ist aus der Gruppe bestehend aus Nieren, Herz, Leber, Lunge, Knochenmark, Pankreas (Inselzellen), Kornea, Dünndarm, Haut und Herzklappen.

4. Verwendung nach einem der Ansprüche 1 bis 3, wobei das Medikament eine Kombination von 29-Demethoxyrapamycin und einem oder mehreren Anti-Abstoßungs-Chemotherapeutika umfaßt.

5. Verwendung nach Anspruch 4, wobei das Anti-Abstoßungs-Chemotherapeutikum ausgewählt ist aus einem oder mehreren von Azathioprin, Corticosteroiden, Cyclophosphamid, Rapamycin, Cyclosporin A, FK-506, OKT-3 und ATG.

6. Verwendung nach einem der Ansprüche 1 bis 5, wobei das Medikament zur oralen, parenteralen, intranasalen, intra-bronchialen, transdermalen oder rektalen Verabreichung geeignet ist.

7. Pharmazeutische Zusammensetzung, welche eine Kombination von 29-Demethoxyrapamycin und einem oder mehreren Anti-Abstoßungs-Chemotherapeutika und einen pharmazeutisch annehmbaren Träger umfaßt.

8. Produkt, welches 29-Demethoxyrapamycin und ein Anti-Abstoßungs-Chemotherapeutikum als kombinierte Zubereitung zur gleichzeitigen, getrennten oder sequentiellen Verwendung bei der Induktion einer Immunsuppression bei einem Säuger enthält.

9. Zusammensetzung oder Produkt nach Anspruch 7 oder 8, wobei das Anti-Abstoßungs-Chemotherapeutikum ausgewählt ist aus Azathioprin, Corticosteroiden, Cyclosporin A, FK-506, OKT-3 und ATG oder einer Kombination beliebiger der vorstehend angegebenen.

## Revendications

1. Utilisation de la 29-déméthoxyrapamycine dans la préparation d'un médicament pour induire l'immunosuppression chez un mammifère.

2. Utilisation de la 29-déméthoxyrapamycine dans la préparation d'un médicament pour prévenir ou traiter le rejet de transplantation d'organe ou de tissu chez un mammifère.

3. Utilisation suivant la revendication 2, dans laquelle l'organe ou le tissu transplanté est choisi parmi le groupe consistant en rein, coeur, foie, poumon, moelle osseuse, pancréas (cellules îlots), cornée, intestin grêle, peau et valve cardiaque.

4. Utilisation suivant l'une quelconque des revendications 1 à 3, dans laquelle le médicament comprend une combinaison de 29-déméthoxyrapamycine et d'un ou de plusieurs agents chimiothérapeutiques antirejets.

5. Utilisation suivant la revendication 4, dans laquelle l'agent chimiothérapeutique antirejet est un ou plusieurs parmi l'azathioprine, les corticostéroïdes, le cyclophosphamide, la rapamycine, la cyclosporine A, le FK-506, l'OKT-3 et l'ATG.

6. Utilisation suivant l'une quelconque des revendications 1 à 5, dans laquelle le médicament est destiné à une administration orale, parentérale, intranasale, intrabronchique, transdermique ou rectale.

7. Composition pharmaceutique qui comprend une combinaison de 29-déméthoxyrapamycine et d'un ou de plusieurs agents chimiothérapeutiques antirejets, et d'un support pharmaceutiquement acceptable.

8. Produit contenant la 29-déméthoxyrapamycine et un ou plusieurs agents chimiothérapeutiques antirejets sous forme d'une préparation combinée pour une utilisation simultanée, séparée ou séquentielle pour induire l'immunosuppression chez un mammifère.

9. Composition ou produit suivant la revendication 7 ou 8, dans lequel l'agent chimiothérapeutique antirejet est choisi parmi l'azathioprine, les corticostéroïdes, la cyclosporine A, le FK-506, l'OKT-3 et l'ATG ou une combinaison des quelconques précédents.
